Europäisches Patentamt

European Patent Office

Office européen des brevets

(19)

(11) Publication number: **0 155 104**
A2

(12) **EUROPEAN PATENT APPLICATION**

(21) Application number: **85301212.8**

(22) Date of filing: **22.02.85**

(51) Int. Cl.⁴: **G 01 N 33/53**, G 01 N 33/78

(30) Priority: **24.02.84 GB 8404843**

(43) Date of publication of application: **18.09.85**
**Bulletin 85/38**

(84) Designated Contracting States: **DE FR GB IT**

(71) Applicant: **AMERSHAM INTERNATIONAL plc, White Lion Road, Amersham Buckinghamshire, HP7 9LL (GB)**

(72) Inventor: **Midgley, John Edward Maurice, 10, Chestnut Close, Amersham Buckinghamshire (GB)**

(74) Representative: **Pennant, Pyers et al, Stevens, Hewlett & Perkins 5 Quality Court Chancery Lane, London, WC2A 1HZ (GB)**

(54) **Free analyte assay.**

(57) In an assay of the free portion of an analyte, e.g. thyroxine, in a biological fluid in which the analyte is present partly bound to natural protein binders, in which the free analyte and an analyte derivative compete for reaction with a specific binder, a differential blocking agent is used to reduce binding of the analyte derivative, but without reducing binding of the analyte, to the natural protein binders. When the natural binder is albumin, the differential blocking agent may have an affinity constant for albumin of about $10^4$ - $10^6$. When the analyte is thyroxine, the differential blocking agent may be 5-sulphosalicyclic acid or a derivative thereof at a concentration of $5 \times 10^{-5}$M to $5 \times 10^{-2}$M.

EP 0 155 104 A2

# Free Analyte Assay

This invention relates to an assay for the free portion of organic substances or analytes that are present in biological fluids in both a form bound to protein (or other binding species present in the fluid) and in a non-bound or free form, the free and bound forms being in equilibrium with one another.

For most physiologically active substances that can be found jointly in both a free form and a protein-bound form in biological fluids such as blood, it is currently thought that it is the concentration of the free form that may control the physiological responses associated with those substances and may therefore be more significant clinically than in the concentration of total substance which includes both free (or unbound) and protein-bound substance.

European Patent Specification No. 26103 describes an assay method of this kind. The method involves causing the analyte and a labelled derivative thereof to compete for reaction with a specific binder for the analyte. The amount of the labelled derivative of the analyte which has become bound to the specific binder is then measured, and the measurement used to determine the concentration of the free analyte in the biological fluid. The amounts of labelled derivative and specific binder used are insufficient to substantially affect the aforesaid equilibrium. Also, the labelled derivative of the analyte is chosen to be substantially non-reactive with the natural binders in the biological fluid.

European Patent Specification 89806 describes a somewhat similar assay, in which however, it is the specific binder rather than the analyte derivative which is labelled. The analyte and its derivative compete for binding with the labelled specific binder. The amount of analyte derivative bound to the labelled specific binder is measured and the measurement used to determine the free analyte concentration. As before, the amounts of analyte derivative and labelled specific binder are insufficient to substantially disturb the free-bound analyte equilibrium; and the analyte derivative is chosen to be substantially non-reactive with the natural binders in the biological fluid.

The present invention is concerned with assays of the kind described in the aforesaid two European Patent Specifications. Development of these assays has been slowed by the difficulty of finding analyte derivatives which meet somewhat exacting requirements. These are:-

a) For use in the assay of European Patent Specification 26103, the analyte derivative must be labelled. For use in the assay of European Patent Specification 89806, the analyte derivative must generally be readily separable, after reaction with the labelled specific binder, from the liquid assay medium.

b) The analyte derivative must compete with the analyte for reaction with the specific binder.

c) The analyte derivative must be substantially non-reactive, and preferably completely non-reactive, with the natural binders for the analyte in the biological fluid.

It is sometimes difficult to devise an analyte derivative which, while binding strongly to the specific binder, is completely non-reactive with natural binders for the analyte in the biological fluid. This applies particularly when the biological fluid contains several different natural binders for the analyte. If an analyte derivative is used which does bind significantly to one of these natural

- 3 -

binders, then assay results will be obtained which, while nominally being measurements of free analyte concentration, do in fact show some correlation with the concentration of that natural binder in the biological fluid.

The present invention is based on the idea of using a differential blocking agent to reduce the extent of binding of the analyte derivative to the natural binders, while not unduly affecting the free-bound analyte equilibrium.

The use of blocking agents in assays for total analyte is well known. The blocking agent is added in order to compete with the analyte for reaction with the natural binders and effectively displaces the fraction of the analyte originally bound to those binders. The analyte is now completely available for binding with a suitable antibody, and is then easily assayed by conventional methods. For this purpose, the blocking agent needs to have a high affinity, as high as possible, for the natural binders and to be used in a concentration sufficient to make all the analyte available for binding with the antibody.

The thyroid hormones may be considered as examples of such analytes. Thyroxine (T4) is present in body fluids 99.97% bound to natural protein binders and only 0.03% in free form. Of the bound fraction, 70% is bound to thyroxine binding globulin (TBG), 20% to thyroxine binding prealbumin (TBPA) and 10% to albumin (Alb). Blocking agents frequently used for total T4 assay include 8-anilino-naphthalene sulphonic acid and thiomersalate. Tri-iodothyronine (T3) is present in body fluids 99.7% bound to natural protein binders and 0.3% in free form. Of the bound fraction, 60% is bound to TBG, 15% to TBPA and 25% to Alb. Sodium salicylate has been used as a blocking agent for both total T3 and total T4 assays.

Unlike these blocking agents used in conventional total analyte assays, the differential blocking agents used in the present invention are intended to displace as little as possible of the analyte initially bound to the natural binders.

The present invention provides a method of determining the concentration of the free portion of an analyte which is a member of a specific binding pair consisting of the analyte and a specific binder therefor, said free portion of the analyte being present in a biological fluid which also contains a portion of the analyte bound to one or more natural binders for the analyte, the bound and free portions of the analyte being in equilibrium with one another, by

a) forming a mixture of a sample of the biological fluid with an amount of a specific binder for the analyte insufficient to substantially affect said equilibrium, and with a derivative of the analyte that has a lower affinity for the natural binders than does the analyte itself, and

b) maintaining the mixture for a time to permit the free portion of the analyte derivative to compete for binding with the specific binder, measuring the amount of the analyte derivative that has, or has not, become bound to the specific binder, and using the measurement to determine the concentration of free analyte in the biological fluid.

characterized in that there is included in the mixture a differential blocking agent in solution, which differential blocking agent has an affinity for at least one natural binder in the biological fluid, and is used in a concentration such that binding of the analyte derivative to the natural binder is substantially reduced without substantially reducing binding of the analyte to the natural binder.

It is essential that the affinity of the analyte for the natural binder be greater than that of the analyte derivative. Given that premise, any blocking agent which competes for binding sites on the natural binder will displace a higher proportion of bound analyte derivative than of bound analyte; i.e. will act as a differential blocking agent. The bigger the difference between the affinities of the analyte and of the analyte derivative for the natural binder, the easier will be the task of achieving the differential blocking. If the difference in affinities is large enough, no differential blocking agent will be needed. The difficulty of finding a suitable analyte derivative, having zero or negligible affinity for the natural binder, creates the need for the use of a differential blocking agent according to this invention.

The differential blocking agent must have a certain affinity for binding with the natural binder. It must also compete with the analyte derivative for binding to the natural binder. In practice, this generally means that the differential blocking agent and the analyte derivative must bind to the same sites, or at least to neighbouring sites, on the natural binder.

The affinity of the differential blocking agent for the natural binder varies inversely with the concentration at which it is included in the reaction mixture. If a differential blocking agent having low affinity is chosen, then a high concentration will need to be included in the mixture. Thus, a lower limit on the affinity of the differential blocking agent is set by its solubility in the liquid assay medium. Correspondingly, if a differential blocking agent having high affinity is chosen, then a low concentration will need to be used.

The need for a free analyte assay, as opposed to a total analyte assay, arises because of the different concentrations of natural binder that may be present in the biological fluid forming the assay sample. So to be useful, the differential blocking agent must be capable of coping with different concentrations of natural binder. This capability varies inversely with the affinity of the differential blocking agent for the natural binder; a high affinity agent could be used in low concentration and the resulting system might not be effective at greatly differing concentrations of natural binder. So, where considerable variations of natural binder concentration are expected, it is preferred that the affinity of the differential blocking agent for the natural binder be not significantly greater than, and desirably less than, the affinity of the analyte for the natural binder.

Once a differential blocking agent having a suitable affinity for the natural binder has been chosen, the next step is to determine how much to use. This is generally done by trial and error. If too much is used, much of the analyte will be displaced, in addition to nearly all the analyte derivative, from the natural binder, and the assay will tend to measure total, rather than free, analyte. If too little is used, not much of the analyte derivative will be displaced from the natural binder, and the assay measurements of free analyte will continue to show some unwanted correlation with natural binder concentration.

As regards the nature of the analyte, the method of this invention is of general applicability. Examples of classes of analyte to which the method may be applied are hormones, biochemical messengers, steroids, drugs, drug metabolites, polypeptides, proteins, vitamins, tumour antigents, toxins, alkaloids and mono-, di- and poly-saccharides. The specific binder, the analyte derivative, the label, and the assay conditions may all be chosen along the lines indicated in the aforesaid European Patent Specifications 26103 and 89806.  In each instance, a suitable differential blocking agent is selected using the criteria outlined above.

Many of these analytes have the property of binding to several of the natural binding proteins in the biological fluid.  Thus, for example, the thyroid hormones bind specifically to TBG and TBPA, and less specifically and with rather lower affinity to albumin.  Cortisol binds specifically to cortisol binding globulin, and less specifically and with rather lower affinity to albumin;  the same is true of other steroids such as progesterone and testosterone.  In fact, many possible analytes show rather non-specific binding to albumin at an affinity constant of around $10^5 - 10^6$.  Although the affinity of the analyte for albumin is lower than for its specialised natural binder, so much albumin is present in the biological fluid that a substantial proportion of the analyte may be bound to it.

Moreover, it is often a matter of particular difficulty to find an analyte derivative that does not bind significantly to albumin.  So the presence of albumin in the sample often represents a major obstacle to a one-step assay for free analyte.  So compounds that bind to albumin at an affinity constant of around $10^4 - 10^6$ are potentially suitable differential blocking agents for use in the method of this invention for measuring a variety of analytes.  Compounds which bind to albumin with this sort of affinity are described in the literature, and include 5-sulphosalicylic acid, salicylic acid, barbitol, gentisic acid, gamma-resorcylic acid and 2,4-dinitrophenol, and derivatives e.g. salts and esters thereof.  These compounds will not necessarily be suitable for the assay of any particular analyte - for example, they may not compete for the same binding sites as the analyte derivative - but they will be worth considering.

Assays of free thyroxine (T4) are now commercially available. This invention is of particular value in relation to such assays. The remainder of this specification is concerned with the commercially available Amerlex (Trade Mark of Amersham International plc) radioimmunoassay kit for measuring free T4 in serum or plasma.

In this kit, the free T4 in the assay sample competes with $^{125}$I-labelled T4 analogue (analyte derivative) for reaction with an immobilised antibody for T4. In this kit, the T4 analogue used as the labelled tracer has a considerably diminished affinity for the thyroid hormone binding sites of TBG, TBPA and Alb, compared with T4 itself. The analogue used in the assay binds with TBG and TBPA at about 3% of the affinity of T4 for these proteins and to albumin at about 20-30% of the affinity of T4. The large difference in affinity of binding of the T4-analogue and T4 itself permits a differential displacement of the analogue and T4 from the serum binding protein, whereby a blocking agent with suitable properties and at a suitable concentration may displace the more weakly bound analogue, whilst scarcely affecting the more strongly bound T4.

Calculations based on the known concentrations of serum T4-binding proteins and the affinity of T4 and the T4-analogue for the binding sites, indicate that albumin-binding of the T4-analogue is a more important factor in any distortion of measured serum free T4 concentrations than equivalent binding to either TBG or TBPA. In agreement with this prediction, it has been shown that, in a group of euthyroid subjects with widely varying serum concentrations of TBG, TBPA and albumin, there is no correlation of free T4 estimates with either TBG or TBPA concentrations. However a small, but significant, correlation between free T4 estimates and albumin concentrations is discernible though this is of no clinical significance. It is therefore of paramount importance to devise a blocking agent that would significantly minimise the effect of residual binding of the T4 analogue to serum albumin in the free T4 assay. In this way, the minor correlation of free T4 concentrations against serum albumin levels would be further reduced. Because of the fact that T4 is only weakly bound to albumin, such a blocker might also displace a small fraction of the hormone from this protein as well as displacing a larger fraction of the still more weakly bound T4-analogue. However, as albumin binding of T4 is only a minor component in the Mass Action equilibrium between bound and free T4 in serum or plasma, a small displacement of T4 on the other more strongly binding proteins (TBG or TBPA) can be tolerated without a significant change in free T4 concentration. Any such minor effects on T4 binding are, additionally, calibrated out by the comparable effects occurring for standards and controls used in the radioimmunoassay of free T4.

As a differential blocking agent for a free T4 assay, 5-sulphosalicylic acid (SSA) (and derivatives e.g. salts and esters thereof) is a suitable choice. Hitherto, the use of this substance has been largely directed as an acid precipitant of proteins (including albumin) from urine in the estimation of precipitable protein in this milieu. There are to the best of our knowledge no reports of its use as a blocking agent against the binding of T4 or T3 to serum binding proteins. Indeed, the increased polarity of the SSA molecule conferred by the sulphonic acid radical should render it less avid than salicylate itself for competitive binding at the largely hydrophobic T4-binding sites of albumin, and the TBG or TBPA T4- binding sites. SSA thus has considerable flexibility in its use as a differential blocker of T4-analogue binding to serum protein binding sites as regards its solubility and affinity.

If a suitable concentration of SSA is used to perform this differential blocking effect, seven predictions can be made to verify that albumin binding of the T4-analogue in the free T4 immunoassay is differentially inhibited.

1) If progressively increasing concentrations of SSA are used in a dialysis experiment, where serum albumin is equilibrated in a dialysis bag with $^{125}$I-labelled T4 or T4-analogue in tracer quantities, the increase in the percentage of tracer, freed from albumin binding and thus dialysed through the membrane of the bag, should be greater for the T4-analogue than for T4, if the analogue is more weakly bound and thus more easily displaced.

2) If the same experiment is performed using a sample of serum with normal TBG, TBPA and albumin concentrations, but only tracer quantities of either $^{125}$I-labelled T4 or T4-analogue, no significant displacement of T4 should be noted, though displacement of the analogue may, at the same SSA concentration, be significantly raised, because of its relatively weak binding.

3) The addition of SSA to the working immunoassay for free T4 should significantly raise the $B_o$ (percentage of counts bound to antiserum at zero T4 concentration) because more labelled T4-analogue should be displaced from serum proteins (especially albumin) and thus become available for binding by the antiserum.

4) The addition of extra albumin to a serum should have less effect in the presence of SSA than in its absence, on the apparent values for serum free T4 obtained by the immunoassay. This is because the extra albumin sequestrates more tracer. removing it from the immunoassay process and thus artifically raising the results. SSA, by opposing this sequestration, will mitigate the distorting effects of added albumin.

5) The artificially low serum free T4 values estimated in an analbuminemic patient should be rectified by the use of SSA, or its congeners. This is because the sequestration of analogue tracer by albumin in serum controls and standards (with normal concentrations of albumin) is minimised by SSA, but of course is, as it always was, absent or strongly reduced in the analbuminemic serum. Compared with the assay standards therefore, the assay process is treating both low (absent)-albumin and normal-albumin sera in a more closely comparable fashion, with predictable results for the analbuminemic serum value, as interpolated from the standard dose-response curve.

6) Non-esterified fatty acids (NEFA) (e.g. oleic acid) bind strongly with sites on serum albumin and, in so doing, displace firstly the residually bound T4 analogue and secondly the more strongly bound T4 from the albumin binding sites. Because of this effect, the release of albumin-bound T4-analogue tracer by NEFA in the free T4 radioimmunoassay at first causes an apparent fall in the free T4 estimation (to 30-40% of the original value in the absence of NEFA), which then rises again on further addition of NEFA, owing to the secondary phenomenon of displacement of the more strongly bound T4 into the free phase.

If SSA is successfully "blocking" albumin binding sites and preventing the labelled T4-analogue from effectively binding , then the addition of NEFA should have a much smaller depressive effect on the free T4 radioimmunoassay results since less tracer is available for displacement.

7) If the principal effect of SSA or its congeners is to block the binding of the labelled T4-analogue to serum albumin binding sites, then sera with TBG concentrations very different from those in the standards or controls, but with normal albumin concentrations should not be affected significantly by the presence of SSA. Nor should the presence of salicylate or phenytoin (as drugs) in serum samples interfere with the effect of SSA, provided always that the concentration of such binders with albumin T4-binding sites is effectively much smaller than that of SSA in the working radioimmunoassay. This is likely to be the case, given that there is only 100 microlitres of serum in a total of 1.1 ml of reactants in the immunoassay process.

The following Examples illustrate the invention and test these predictions.

### Example 1

A stock solution of 5-sulpho-salicylic acid was made up in water (and neutralised to pH 7.4 with sodium hydroxide) such that dilution of this solution by a factor 1:8 gave a final concentration of $5 \times 10^{-2}$M. High specific activity $^{125}$I-labelled T4-analogue (a complex of T4 with EDTA) was diluted in buffer containing 0.2M phosphate, 0.1M EDTA, and 0.1M sodium azide (pH 7.4) to yield a total of approximately 250,000 cpm in each experiment.

Various mixtures of SSA solution, $^{125}$I-T4 analogue solution and buffer were added to a fixed concentration of the "zero" standard (containing no endogenous T4) of the Amerlex $^{(R)}$ Free T4 radioimmunoassay kit to produce a final volume of 4 ml. These solutions were placed in dialysis bags and were dialysed against 4 ml of phosphate, EDTA, azide buffer pH 7.4, placed in contact with the bag, overnight at room temperature. Table 1 shows the various solutions made for this experiment.

- 13 -

Table 1

| | SSA | | Inside Dialysis Bag | | | Outside Dialysis Bag |
| Vol (ml) | Concn. (M) | T4 Analogue (ml) | Zero Standard (ml) | Buffer (ml) | Buffer (ml) |
|---|---|---|---|---|---|
| 1 | 5x10-2 | 0.5 | 0.4 | 2.1 | 4.0 |
| 0.4 | 2x10-2 | 0.5 | 0.4 | 2.7 | 4.0 |
| 0.1 | 5x10-3 | 0.5 | 0.4 | 3.0 | 4.0 |
| 0.01 | 5x10-4 | 0.5 | 0.4 | 3.1 | 4.0 |
| 0.001 | 5x10-5 | 0.5 | 0.4 | 3.1 | 4.0 |

A similar experiment was carried out, except that human serum albumin was used instead of the zero standard of the kit, the concentration of albumin within the dialysis bag being 40 g/1.

Experiments corresponding to the two described above were also performed for tracer quantities of $^{125}$I-labelled T4 at high specific activity. In all cases, aliquots of solution from the inside and outside of the dialysis bag were taken for counting after equilibration had been reached and the proportion of protein-bound and free (unbound) tracer was calculated as follows:-

Counts/min. inside bag =
Fraction Bound (B) + ½ Fraction Free
Counts/min. outside bag =
½ Fraction Free,

since the solution volumes inside and outside the bag are equal. Therefore the percentage of protein bound T4 or T4-analogue is given by:

$$100 \cdot \frac{(\text{Counts/min inside bag} - \text{Counts/min outside bag})}{(\text{Total Counts/min. inside and outside bag})}$$

The results for T4-analogue and T4 are given in Tables 2 and 3.

### Table 2
### Displacement of $^{125}$I-labelled T4 analogue
### or T4 from serum albumin

| SSA Concn.(M) | Percentage of tracer bound T4 Analogue | T4 |
|---|---|---|
| 5x10-2 | 62 | 83 |
| 2x10-2 | 70 | 85 |
| 5x10-3 | 75 | 90 |
| 5x10-4 | 80 | 92 |
| 5x10-5 | 83 | 93 |
| 0 | 90 | 95 |

### Table 3
### Displacement of $^{125}$I-labelled T4-analogue
### or T4 from proteins of the zero standard

| SSA Concn. (M) | Percentage of tracer bound T4 analogue | T4 |
|---|---|---|
| $5 \times 10^{-2}$ | 75 | 95 |
| $2 \times 10^{-2}$ | 79 | 95 |
| $5 \times 10^{-3}$ | 83 | 93 |
| $5 \times 10^{-4}$ | 89 | 96 |
| $5 \times 10^{-5}$ | 90 | 97 |
| 0 | 90 | 96 |

These tables show that for both albumin and the zero standard, T4-analogue is more readily displaced from protein binding sites by SSA than is T4. Indeed T4 binding by the zero standard is not affected over the range of SSA concentrations used, whereas some of the analogue is displaced at SSA concentrations greater than $5 \times 10^{-4}$M. A considerably larger fraction of the analogue is displaced from albumin at concentrations of SSA greater than $5 \times 10^{-4}$M, whereas, up to $2 \times 10^{-2}$M, T4 displacement is small (Table 2).

These experiments demonstrate that SSA at suitable concentrations displays differential blocking of T4 and its analogue from both albumin and possibly other serum protein T4-binding sites. Concentrations of SSA can be chosen where T4 displacement is minimal.

Example 2

The effect of increasing concentrations of SSA on the value for Bo (the percentage of T4-analogue tracer binding to antiserum at zero T4 concentration) is shown in Table 4.

Table 4

| SSA Concn. (M) | Bo (% tracer bound) |
|---|---|
| $2 \times 10^{-2}$ | 82 |
| $1 \times 10^{-2}$ | 82.5 |
| $5 \times 10^{-3}$ | 78 |
| $2 \times 10^{-3}$ | 80 |
| $1 \times 10^{-3}$ | 74 |
| $5 \times 10^{-4}$ | 72 |
| $2 \times 10^{-4}$ | 68 |
| $5 \times 10^{-5}$ | 66 |
| 0 | 65 |

As expected from prediction number 3, the SSA, by differentially blocking T4-analogue binding by serum proteins, permits more tracer to be available for binding to the antiserum and thus raises the Bo value.

## Example 3

To aliquots of a euthyroid serum pool, bovine serum albumin (crystalline) was added to various concentrations. For each concentration of added albumin, various concentrations of SSA were added to sub-aliquots to test the counter-effect of SSA on distortions of free T4 estimations by the Amerlex kit caused by the added albumin. The results are shown in Table 5.

### Table 5

| Concn. of added albumin g/l | (Percentage of original value) FT4 value by kit | | |
|---|---|---|---|
| | NoSSA | SSA $10^{-3}$M | SSA $3 \times 10^{-3}$M |
| (NB albumin in serum originally at 40g/l | | | |
| 0 | 100 | 100 | 100 |
| 10 | 118 | 114 | 118 |
| 20 | 144 | 120 | 125 |
| 40 | 189 | 150 | 146 |
| 80 | 277 | 207 | 224 |

As expected by prediction number 4, the addition of SSA at $10^{-3}$M or above reduces the distorting effects of sequestration of tracer by extra added albumin by about 30%. This shows the SSA has limited the

- 17 -

sequestration of tracer by albumin as expected.  It must be remembered that as the SSA/added albumin ratio falls, the effective blocking power of the SSA is also progressively reduced.  Further, concentrations of albumin above 70 g/l (40 g/l endogenous + 30 g/l added) are probably non physiological.

## Example 4

A serum sample from an analbuminaemic patient (albumin 4 g/l) with suspected borderline hypothyroidism - borderline TSH value and clinical signs and symptoms, was measured for serum free T4 in a standard Amerlex free T4 assay in the presence and absence of added SSA,  Table 6 shows the results.

### Table 6

| SSA<br>Concn. (M) | FT4 value (pmol/l) |
|---|---|
| $5 \times 10^{-2}$ | 9.4 |
| $3 \times 10^{-3}$ | 11.6 |
| $10^{-3}$ | 10.4 |
| 0 | 7.1 |
| Theoretical | 11.0 |

As forecast in prediction number 5, the Free T4 estimation for the analbuminaemic patient is raised by the addition of SSA close to that predicted both from a knowledge of the total T4, TBG, TBPA and albumin concentration in this serum and the borderline hypothyroid status of the subject.  The lower limit of the normal range in the kit is 8.5 pmol/l.  Without SSA, the free T4 value is clearly hypothyroid, but is typical of borderline patients in the presence of SSA at $10^{-3}$M or above.

## Example 5

Oleic acid was added at various concentrations to a pool of euthyroid serum. To aliquots of this pool, SSA was also added at progressively increasing concentrations. The results are shown in Table 7.

### Table 7
#### Effect of SSA in mitigating the distortion of free T4 results occasioned by Non Esterified Fatty Acids (NEFA)

| Oleic Acid Concn. (mM/l) | S.S.A. Concn. (M) | | | | | | |
|---|---|---|---|---|---|---|---|
| | 0 | $10^{-3}$ | $2 \times 10^{-3}$ | $5 \times 10^{-3}$ | $10^{-2}$ | $2 \times 10^{-2}$ | $5 \times 10^{-2}$ |
| 0 | 100 | 100 | 100 | 100 | 100 | 100 | 100 |
| 1.25 | 82.8 | 94.4 | 87.4 | 106 | 90.4 | 85.8 | 82.1 |
| 2.5 | 68.2 | 86.1 | 83.5 | 83.2 | 90.4 | 75.2 | 74.0 |
| 5 | 48.4 | 77.0 | 74.0 | 75.2 | 72.1 | 66.7 | 61.8 |
| 10 | 48.4 | 88.1 | 91.3 | 92.8 | 82.4 | 78.0 | 70.0 |
| 12.5 | 62.4 | 110 | 105.5 | 92.0 | 98.5 | 101.4 | 81.3 |
| 15 | 73.2 | 118 | 113.4 | 101.6 | 123.5 | 129.1 | 104.1 |
| 20 | 118.5 | 285 | 188.2 | 160.8 | 186.0 | 202.1 | 143.9 |

The free T4 values are expressed as a percentage of values for each SSA concentration of zero oleic acid concentration (100%).

As expected, from prediction number 6, the addition of SSA mitigated the effect of oleic acid (NEFA) in the displacement of T4 analogue tracer from the serum binding proteins. The reason is as described under this prediction. Optimum SSA effects (i.e. a minimum oleic acid effect at 5mM/l) are found at about $10^{-3}$ SSA. All SSA levels of $10^{-3}$ or above have significant effects in this repsect. The reason for the slightly worse performance of higher SSA concentrations is unclear.

Example 6

Serum free T4 measurements were made by the Amerlex[(R)] free T4 RIA kit on sera with normal albumin concentration, but with unusually high or low TBG concentrations, in the presence of SSA.  A concentration of $10^{-3}$M SSA was chosen as optimum (from experience in the previous examples).

Table 8

| Serum sample | Free T4 value (pmol/l) | |
|---|---|---|
| | No SSA | $10^{-3}$M SSA |
| Zero TBG Euthyroid | 18.1 | 21.7 |
| High TBG | 19.3 | 19.8 |
| Euthyroid on Aspirin Therapy | 19.4 | 19.7 |
| Euthyroid on Phenytoin Therapy | 12.0 | 11.5 |
| Normal Range = 8.5 - 26 pmol/l | | |

There was no significant effect of SSA on free T4 values in these sera, as forecast by prediction 7.

Example 7

Other compounds, described alongside SSA in the literature as being capable of displacing T4-binding proteins (e.g. thyroxine binding prealbumin), were also tested for their ability to act as differential blocking agents in the FT4 assay.  A useful differential blocking agent must demonstrate progressively increasing Bo values in the FT4 assay (see Table 4), as the concentration of the putative blocker is increased.  Table 9 shows the results for additions of i) sodium salicylate, ii) sodium gentisate (2,5-dihydroxybenzoate), iii) sodium gamma-resorcylate

- 20 -

(2,6-dihydroxybenzoate), iv) salicylamide, and v) 2,4-dinitrophenol at concentrations of $10^{-2}$ and $10^{-3}$M.

Table 9

| Substance used as blocking agent | Concentration (M) | Bo (% tracer bound) |
|---|---|---|
| Salicylate | $10^{-2}$ | 72.6 |
| | $10^{-3}$ | 69.5 |
| Gentisate | $10^{-2}$ | 71.8 |
| | $10^{-3}$ | 70.5 |
| Gamma resorcylate | $10^{-2}$ | 51.6 |
| | $10^{-3}$ | 70.3 |
| Salicylamide | $10^{-2}$ | 43.0 |
| | $10^{-3}$ | 67.5 |
| 2,4-dinitrophenol | $10^{-2}$ | - |
| | $10^{-3}$ | 60.0 |
| No addition | - | 67.7 |

From the Table, it is clear that neither gamma-resorcylic acid, salicylamide nor 2,4-dinitrophenol are of value as differential blocking agents using the particular assay described in this example, but both sodium salicylate and sodium gentisate show some degree of displacement of labelled analogue from the serum proteins. They are less effective in this particular instance than SSA (Figure 4), but would probably be more effective in displacing an analogue of weaker affinity with the serum protein binding sites. These experiments demonstrate the essentially empirical nature of the method needed to determine the suitability of a particular differential blocker for optimal effects on a particular immunoassay, but show that a whole range of such differential blockers is possible, with widely differing affinities for the serum protein binding sites and therefore suitable for a wide range of analyte assays, depending on the absolute and relative binding affinities of the analyte and its analogue in any one case.

0155104

- 22 -

C L A I M S

1. A method of determining the concentration of the · free portion of an analyte which is a member of a specific binding pair consisting of the analyte and a specific binder therefor, said free portion of the analyte being present in a biological fluid which also contains a portion of the analyte bound to one or more natural binders for the analyte, the bound and free portions of the analyte being in equilibrium with one another, by

    a) forming a mixture of a sample of the biological fluid with an amount of a specific binder for the analyte insufficient to substantially affect said equilibrium, and with a derivative of the analyte that has a lower affinity for the natural binders than does the analyte itself, and

    b) maintaining the mixture for a time to permit the free portion of the analyte derivative to compete for binding with the specific binder, measuring the amount of analyte derivative that has, or has not, become bound to the specific binder, and using the measurement to determine the concentration of free analyte in the biological fluid.

    characterized in that there is included in the mixture a differential blocking agent in solution, which differential blocking agent has an affinity for at least one natural binder in the biological fluid, and is used in a concentration such that binding of the analyte derivative to the natural binder is substantially reduced without substantially reducing binding of the analyte to the natural binder.

2. A method as claimed in claim 1, wherein the affinity of the differential blocking agent for the natural binder is not significantly greater than the affinity of the analyte for the natural binder.

3. A method as claimed in claim 1 or claim 2, wherein the natural binder is albumin and the differential blocking agent has an affinity constant of around $10^4$ - $10^6$ for albumin.

4. A method as claimed in claim 3, wherein the differential blocking agent is selected from 5-sulphosalicylic acid, salicylic acid, barbitol, gentisic acid, gamma-resorcylic acid and 2,4-dinitrophenol, and derivatives thereof.

5. A method as claimed in any one of claims 1 to 3, wherein the analyte is a thyroid hormone.

6. A method as claimed in claim 5, wherein the analyte is thyroxine.

7. A method as claimed in claim 6, wherein the differential blocking agent is 5-sulphosalicylic acid or a derivative thereof.

8. A method as claimed in claim 7, wherein the differential blocking agent is used at a concentration of from $5 \times 10^{-5}$ M to $5 \times 10^{-2}$ M.